# FASCICULE DE BREVET EUROPEEN

(11) **EP 1 293 502 B1**
(45) Date de publication et mention de la délivrance du brevet: **09.05.2007**
(21) Numéro de dépôt: 02292058.1
(22) Date de dépôt: 20.08.2002
(51) Int. Cl.: C07D 233/32

(54) **Procédé de préparation de (meth)acrylates d'alkylimidazolidone**
Verfahren zur Herstellung von (Meth)acrylaten von Alkylimidazolidonen
Process for the preparation of (meth)acrylates of alkylimidazolidone

(30) Priorité: 28.08.2001 FR 0111178
(43) Date de publication de la demande: 19.03.2003
(73) Titulaire: Arkema France, 92800 Puteaux (FR)
(72) Inventeur: Paul, Jean-Michel, 57070 Metz (FR); Dupont, Bernard, 57150 Creutzwald (FR)
(74) Mandataire: Hirsch & Associés

(56) Documents cités:
- EP-A- 0 236 994
- EP-A- 0 447 141
- EP-A- 0 571 851
- EP-A- 0 619 309
- EP-A- 0 712 846
- EP-A- 0 902 017
- EP-A- 0 968 995

## Description

La présente invention a trait à un procédé de préparation de (méth)acrylates d'alkylimidazolidone.

Les acrylates et méthacrylates d'alkylimidazolidone sont connus pour leur rôle dans la constitution de polymères utilisables comme revêtements et adhésifs, dans le traitement du papier et des textiles, pour leur utilisation comme agents de traitement du cuir et dans la production de peintures en émulsion.

La demande de brevet européen n° 236 994 se rapporte à la préparation d'esters de l'acide (méth)acrylique par réaction d'un (méth)acrylate d'alkyle avec un alcool hétérocyclique en présence d'un catalyseur qui est un alcoolate de titane ou un chélate de titane, de zirconium de fer ou de zinc, combiné à un composé 1,3-dicarbonylé, qui peut être un chélate métallique d'une 1,3-dicétone, en particulier un acétylacétonate.

Un tel catalyseur présente l'inconvénient majeur de générer des produits fortement colorés.

La demande de brevet européen n° 433 135 a trait à un procédé de synthèse d'un (méth)acrylate d'alkylimidazoline, dans lequel on fait réagir un (méth)acrylate d'alkyle avec un alcool hétérocyclique en présence d'un catalyseur choisi parmi les dialkyloxydes d'étain, les dialkyldialcoxydes d'étain et les dialkyldiesters d'étain.

Un tel catalyseur nécessite des températures de réaction élevées et entraîne souvent des polymérisations violentes avec formation d'un polymère expansé très dur.

La demande de brevet européen n° 453 638 concerne également la synthèse d'un (méth)acrylate d'alkylimidazoline en présence de dialkyloxyde d'étain.

La demande de brevet européen n°571 851 a pour objet un procédé de préparation de (méth)acrylate d'alkylimidazoline, par transestérification d'un (méth)acrylate d'alkyle au moyen d'un alcool hététocyclique, en présence d'un système catalytique qui est une combinaison d'un catalyseur à base de lithium, par exemple sous forme d'oxyde, d'alcoolate, d'acétate, de chlorure, de bromure, et d'un catalyseur à base de calcium tel que de l'oxyde de calcium.

La demande de brevet européen n° 619 309 se rapporte à la préparation de (méth)acrylate d'alkylimidazoline par réaction d'un (méth)acrylate d'alkyle avec un alcool hétérocyclique en présence d'un catalyseur choisi parmi les chélates de calcium avec des composés 1,3-dicarbonyle, tels que l'acétylacétonate de calcium. Le produit obtenu par le procédé décrit dans ce document présente une coloration trop forte.

La demande de brevet européen n° 447 141 a pour objet un procédé de préparation d'un ester d'acide carboxylique, par transesterification d'un alkylester par un alcool derivé d'un alkyle supérieur, en présence d'un chélate de hafnium comme catalyseur.La réaction procure des rendements élevés.

La demande de brevet européen n° 712 846 a pour objet un procédé de préparation de (méth)acrylates d'alkylimidazolidones par réaction d'un (méth)acrylate avec un alcool hétérocyclique, en présence d'un catalyseur formé d'un mélange d'au moins un alcoolate de magnésium et d'un composant pouvant être notamment un chélate de calcium avec des composés 1,3-dicarbonylés. Le catalyseur employé permet d'obtenir une productivité normale à des températures de réaction plus basses que dans les procédés précédement connus.

Enfin, la demande de brevet européen n° 902 017 mentionne l'utilisation d'un catalyseur qui peut être du lithium, du carbonate de lithium ou de l'hydroxyde de lithium dans la préparation d'un monomère à partir, notamment, d'hydroxyéthyl oxazolidine et de méthacrylate de méthyle.

Cependant, l'utilisation de tels systèmes catalytiques ne permet pas d'atteindre simultanément les objectifs principaux suivants :
- une conversion élevée de l'alcool hétérocyclique ;
- une basse teneur en sous-produits ;
- une cinétique élevée ;
- un niveau thermique bas ; et
- une faible coloration du produit final.

L'invention a donc pour objet un procédé de préparation de (méth)acrylate d'alkylimidazoline, procédé qui permet d'atteindre les objectifs évoqués précédemment.

Précisément, l'invention a pour objet un procédé de préparation d'un composé de formule : 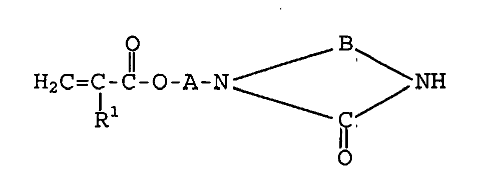 dans laquelle :
- R¹ est un atome d'hydrogène ou un groupement méthyle ; et
- A et B représentent, indépendamment l'un de l'autre, un groupement alkylène à chaîne droite ou ramifiée ayant de 2 à 5 atomes de carbone ;
par réaction d'au moins un (méth)acrylate (II) de formule : 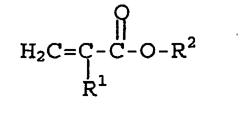 dans laquelle :
- R¹ a la signification précitée ; et
- R² est un groupement alkyle à chaîne droite ou ramifiée ayant de 1 à 4 atomes de carbone ;
avec un alcool hétérocylique (III) de formule : 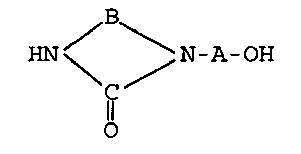 dans laquelle A et B ont les significations précitées ;
en présence d'un catalyseur comprenant au moins un composé (a), qui est un chélate formé de lithium et d'un composé 1,3-dicarbonyle.

L'invention a également pour objet une composition comprenant le composé (I) obtenu par le procédé selon l'invention en solution dans le (méth)acrylate (II).

Enfin, l'invention a aussi pour objet l'utilisation de la composition précitée dans la préparation de polymères utilisables comme revêtements et adhésifs, dans le traitement du papier et des textiles, comme agent de traitement du cuir et dans la production de peintures à caractéristiques d'adhésion humide élevées.

### EXPOSE DETAILLE DE L'INVENTION

Le procédé de préparation selon l'invention, tel qu'il a été défini ci-dessus, fait usage d'un catalyseur comprenant au moins un composé (a) qui est un chélate formé de lithium et d'un composé 1,3-dicarbonyle.

Comme exemples de composés 1,3-dicarbonyle, on peut citer :
- un ester d'acide β-cétonique, comme l'ester acétylacétique ;
- une 1,3-dicétone, comme l'acétylacétone, la 3-méthylacétylacétone, la benzoylacétone, le dibenzoylméthane, la 2,4-hexanedione, la 3,5-heptanedione, la 3-phénylacétylacétone, la 4,4,4-trifluoro-l-phényl-1,3-butanedione, la 2,2,6,6-tétraméthyl-3,5-heptanedione, la 1,1,1-trifluoro 5,5-diméthyl-2,4-hexanedione et la 1,1,1-trifluoro-2,4-pentane-dione.

On préfère tout particulièrement comme composé (a), l'acétyl-acétonate de lithium.

A titre d'exemples de réactif (II), on peut citer les acrylates et méthacrylates de méthyle, d'éthyle, de n-propyle, d'isopropyle, de n-butyle et d'isobutyle.

De préférence, le réactif (II) est le méthacrylate ou l'acrylate de méthyle.

Comme exemple de réactif (III), on peut mentionner la 1-(2-hydroxyethyl)-imidazolidyl-2-one.

La quantité de composé (a) utilisée pour la mise en oeuvre du procédé selon l'invention est habituellement définie par rapport à la quantité d'alcool hétérocylique.

Ainsi, le rapport molaire du composé (a) à l'alcool hétérocyclique (III) est généralement compris entre 0,05 et 0,3% et de préférence entre 0,05 et 0,15%.

Selon une variante avantageuse du procédé selon l'invention, le composé (a) n'est pas utilisé seul comme catalyseur, mais en mélange avec un deuxième composé (b) que l'on peut choisir parmi les alcoolates de magnésium, les alcoolates de sodium, les alcoolates de calcium, l'acétyl-acétonate de calcium, l'oxyde de calcium et l'hydroxyde de calcium.

Comme alcoolates, on peut citer les méthylates, éthylates, propylates, isopropylates, butylates et isobutylates.

En ce qui concerne la quantité de composé (b), elle peut être inférieure à celle du composé (a).

En général, on choisit la quantité de composé (b) de telle sorte que le rapport molaire du composé (b) à l'alcool hétérocyclique (III) soit compris entre 0,01 et 0,1 % et de préférence entre 0,01 et 0,05%.

La réaction entre le réactif (II) et le réactif (III) est généralement mise en mise en oeuvre en présence d'un excès de l'un ou l'autre de ces réactifs.

Il est cependant préférable d'utiliser plus de réactif (II) que de réactif (III).

Ainsi, il est souhaitable que le rapport molaire du (méth)acrylate (II) à l'alcool hétérocyclique (III) soit supérieur à 1, de préférence compris entre 1,1 et 7, en particulier entre 2 et 5.

De cette manière, on obtient à la fin de la réaction une composition comprenant une solution du composé (I) dans le (méth)acrylate (II), par exemple :
- une solution d'acrylate d'éthyl-1-imidazolidyl-2-one dans l'acrylate de méthyle ; ou bien
- une solution de méthacrylate d'éthyl-1-imidazolidyl-2-one dans le méthacrylate de méthyle.

Une telle composition, qui présente une coloration inférieure à 100 APHA, peut avantageusement être utilisée directement dans certaines applications telles que la fabrication de peintures à caractéristiques d'adhésion humide élevées, le traitement du cuir ou la fabrication de revêtements.

Le procédé selon l'invention peut être mis en oeuvre en présence d'un ou plusieurs inhibiteurs de polymérisation, que l'on peut choisir parmi la phénotiazine, l'hydroquinone, l'éther monométhylique d'hydroquinone, le diterbutyl para-crésol, la paraphénylène diamine, le TEMPO (2,2,6,6-tétraméthyl-1-piperidinyloxy) et le di-tertiobutylcatéchol.

Ces composés peuvent éventuellement être associés à des chélatants de métaux tels que l'EDTA, l'acide oxalique ou les produits commercialisés sous la marque VERSENEX.

La teneur en inhibiteur de polymérisation est généralement comprise entre 0,05 et 0,5%, ce pourcentage étant exprimé en poids d'inhibiteur (auquel s'ajoute, le cas échéant, le poids du ou des chélatants associés) par rapport au poids d'alcool hétérocyclique (III).

Pour ce qui est des conditions opératoires, le procédé est habituellement mis en oeuvre à une température comprise entre 70 et 120°C et de préférence entre 85 et 100°C, en milieu liquide.

La pression est en général au plus égale à 1 bar, elle est généralement comprise entre 0,3 et 1 bar.

Avantageusement, la réaction est effectuée sous un bullage d'air.

La durée de la réaction est fonction des conditions opératoires et de l'activité du catalyseur. Elle est généralement comprise entre 6 et 11 heures.

La réaction est souhaitablement mise en oeuvre en milieu anhydre, afin d'éviter la désactivation des catalyseurs.

L'eau présente dans les réactifs est de préférence éliminée par distillation sous forme d'un azéotrope de (méth)acrylate (II) et d'eau.

Le mode opératoire peut être décrit grossièrement comme suit.

On chauffe à reflux le mélange des réactifs (II) et (III) et du ou des inhibiteurs, tout en ajustant la pression dans l'installation afin que la température dans le réacteur se maintienne entre 85 et 100°C.

La stabilisation de la colonne est effectuée en introduisant un ou plusieurs inhibiteurs de polymérisation en tête de colonne, dans le reflux.

L'eau présente dans les réactifs (II) et (III) et l'inhibiteur est éliminée par distillation sous forme d'un azéotrope avec le (méth)acrylate (II).

On introduit ensuite le ou les catalyseurs.

La réaction s'effectue avec génération de l'alcool R²OH, lequel est éliminé par formation d'un azéotrope avec le (méth)acrylate (II), afin de déplacer l'équilibre de la réaction de transestérification.

Ainsi, l'équilibre de la réaction de transestérification est déplacé dans le sens de la formation du composé (I) .

Pendant toute la durée de la réaction, on ajuste la pression afin de maintenir la température à l'intérieur du réacteur entre 85 et 100°C.

Si nécessaire, on fait un apport de catalyseur(s) en cours de réaction.

Lorsqu'il ne se forme plus d'alcool R²OH, la réaction peut être considérée comme terminée.

Le produit brut obtenu est alors refoidi et extrait du réacteur. Il se présente généralement sous la forme d'un liquide limpide et très peu coloré (coloration inférieure à 100 APHA).

L'excès éventuel de (méth)acrylate peut ensuite être éliminé par évaporation (opération dite de strippage), de manière à isoler le composé (I) du milieu réactionnel, généralement à l'état solide : ainsi, l'acrylate d'éthyl-1-imidazolidyl-2-one est un solide blanc cristallin de température de fusion égale à 43°C, soluble à froid dans les cétones, les alcools, les hydrocarbures aromatiques et l'eau, insoluble à froid dans les hydrocarbures saturés et qui précipite à 0°C dans l'acrylate d'éthyle. Le méthacrylate d'éthyl-1-imidazolidyl-2-one est un solide blanc cristallin de température de fusion égale à 47°C, possédant les mêmes propriétés de solubilité que le précédent. A l'issue de l'opération de strippage, le produit solide cristallin peut en outre être purifié par lavage par un alcool léger tel que le méthanol et/ou par un éther de pétrole, puis filtration et séchage.

L'isolement du composé (I) peut aussi être réalisé par strippage partiel du (méth)acrylate (II) puis cristallisation à une température suffisamment basse (de préférence inférieure ou égale à 0°C) et pendant une durée suffisamment longue (pouvant atteindre jusqu'à 15 heures), puis filtration suivie des étapes de purification décrites ci-dessus.

Enfin, une autre méthode pour isoler le composé (I) de la solution le contenant consiste à effectuer une extraction par l'eau, suivie d'une décantation, d'un strippage du (méth)acrylate et des étapes de purification décrites ci-dessus.

### Exemples

Les exemples suivants illustrent la présente invention.

Les abréviations suivantes y sont utilisées :
MAM : méthacrylate de méthyle
HEIO : 1-(2-hydroxyéthyl)-imidazolidyl-2-one
MEIO : méthacrylate d'éthyl-l-imidazolidyl-2-one
EMHQ : éther monométhylique d'hydroquinone
PTZ : phénothiazine
Li(acac) : acétylacétonate de lithium
Ca(acac)₂ : acétylacétonate de calcium
DBTO : dibutyloxyde d'étain

### Exemples 1 à 3

Dans un réacteur en acier inoxydable de 630 litres à double enveloppe alimentée par un thermofluide, équipé d'un agitateur mécanique et surmonté d'une colonne à distiller (diamètre 250 mm, hauteur 4 m) remplie d'un garnissage PALL et surmontée d'une tête de reflux, on introduit :
150 kg de HEIO ;
461 kg de MAM ; et
0,2 kg de PTZ (inhibiteur de polymérisation).

La colonne est stabilisée par introduction dans le reflux d'une solution d'EMHQ à 0,1% en poids dans le MAM.

Le mélange réactionnel est séché par distillation azéotropique de l'eau contenue dans les réactifs (l'azéotrope se formant entre le MAM et l'eau) sous une pression de 400 mm Hg (la température en tête de colonne est de 77°C).

Lorsque l'étape de séchage est terminée, on intoduit le ou les catalyseurs et on ajoute un complément de méthacrylate de méthyle afin de maintenir le rapport molaire MAM/HEIO à sa valeur initiale de 4/1.

Pendant toute la durée de la réaction, on ajuste la pression afin de maintenir la température dans le réacteur à 85°C.

Le méthanol formé par la réaction est éliminé sous forme d'un azéotrope avec le MAM.

La réaction est terminée lorsque la température en tête de colonne ne redescend plus vers celle de l'azéotrope, en reflux total, à la pression de service.

Le brut est alors refroidi et analysé par HPLC.

La coloration du brut est un facteur important. Elle doit être aussi faible que possible.

Les exemples 1 à 3 du Tableau A suivant ont tous été effectués dans les conditions ci-dessus en faisant varier la nature du ou des catalyseurs. L'exemple 2, en dehors du cadre de l'invention, a été effectué à des fins de comparaisons : le catalyseur ne contient pas de composé (a) .

On voit donc que les rendements en MEIO sont comparables pour les exemples 1 à 3. Néanmoins, le catalyseur de l'exemple comparatif 2 donne un produit final ayant une forte coloration.

| TABLEAU A | | | | | | |
|---|---|---|---|---|---|---|
| Exemples | Catalyseur et % molaire par rapport à l'HEIO | Durée de la réaction en heures | Coloration du brut en APHA | Composition du brut (% massiques) | | |
| | | | | MAM % | HEIO % | MEIO % |
| 1 | Li(acac) : 0,15 | 10 | 41 | 57,8 | 1,2 | 33,3 |
| 2 (comparatif) | Ca(acac)₂ : 0,15 | 10 | 100 | 58,7 | 1,4 | 35,7 |
| 3 | Li (acac) et Ca(acac)₂ 0,15 et 0,10 (respectivement) | 8 | 74 | 58,4 | 1,1 | 34,5 |

### Exemples 4 à 9

Des essais ont été effectués dans un petit réacteur en verre à double enveloppe, agité mécaniquement et surmonté d'une colonne à distiller en verre, à garnissage « multiknit » (efficacité de 8 plateaux théoriques) et munie d'une tête de reflux.

Les conditions générales sont identiques à celles des exemples 1 à 3, à l'exception près du rapport molaire MAM/HEIO qui est maintenant de 3,5/1 (au lieu de 4/1 précédemment).

Les exemples 5, 6 et 7, en dehors du cadre de l'invention, ont été effectués à des fins de comparaison : leurs catalyseurs ne contiennent pas de composé (a).

Les résultats sont consignés dans le Tableau B suivant.

On voit donc que les catalyseurs des exemples comparatifs 5, 6 et 7 dépourvus de composé (a) donnent un produit final très coloré.

| TABLEAU B | | | | | | |
|---|---|---|---|---|---|---|
| Exemples | Catalyseur et % molaire par rapport à l'HEIO | Durée de la réaction en heures | Coloration du brut en APHA | Composition du brut (% massiques) | | |
| | | | | MAM % | HEIO % | MEIO % |
| 4 | Li(acac) : 0,2 | 7,5 | 50 | 50,2 | 1,0 | 40,8 |
| 5 (comparatif) | DBTO : 1,3 | 15 | 150 | 52,9 | 0,6 | 49,6 |
| 6 (comparatif) | Zr(acac)₂ 0,2 | 8 | 120 | 51,5 | 0,3 | 44,5 |
| 7 (comparatif) | Ca(acac)₂ : 0,2 | 6 | 110 | 51,8 | 1,7 | 38,7 |
| 8 | Li(acac) et Ca(acac)₂ 0,2 et 0,1 (respectivement) | 6,5 | 70 | 50,7 | 1,2 | 40,2 |
| 9 | Li(acac) et Mg(OEt)₂ 0,2 et 0,1 (respectivement) | 7 | 80 | 50,2 | 1,8 | 39,2 |

## Revendications

1. Procédé de préparation d'un composé (I) de formule : 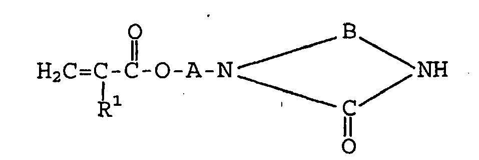 dans laquelle :
- R¹ est un atome d'hydrogène ou un groupement méthyle ; et
- A et B représentent, indépendamment l'un de l'autre, un groupement alkylène à chaîne droite ou ramifiée ayant de 2 à 5 atomes de carbone ;
par réaction d'au moins un (méth)acrylate (II) de formule : 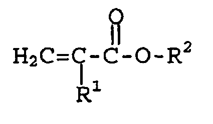 dans laquelle :
- R¹ a la signification précitée ; et
- R² est un groupement alkyle à chaîne droite ou ramifiée ayant de 1 à 4 atomes de carbone ;
avec un alcool hétérocyclique (III) de formule : 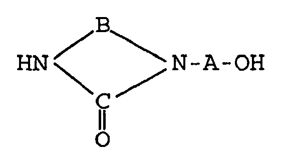 dans laquelle A et B ont les significations précitées ;
en présence d'un catalyseur comprenant au moins un composé (a), qui est un chélate formé de lithium et d'un composé 1,3-dicarbonyle.

2. Procédé selon la revendication 1, dans lequel, le composé 1,3-dicarbonyle est :
- un ester d'acide β-cétonique, comme l'ester acétylacétique ; ou
- une 1,3-dicétone, comme l'acétylacétone, la 3-méthylacétylacétone, la benzoylacétone, le dibenzoylméthane, la 2,4-hexanedione, la 3,5-heptanedione, la 3-phénylacétylacétone, la 4,4,4-trifluoro-1-phényl-1,3-butanedione, la 2,2,6,6-tétraméthyl-3,5-heptanedione, la 1,1,1-trifluoro 5,5-diméthyl-2,4-hexanedione et la 1,1,1-trifluoro-2,4-pentane-dione.

3. Procédé selon la revendication 2, **caractérisé en ce que** le composé (a) est l'acétyl-acétonate de lithium.

4. Procédé selon la revendication 3, **caractérisé en ce** le réactif (II) est de l'acrylate ou du méthacrylate de méthyle et le réactif (III) est la 1-(2-hydroxyéthyl)-imidazolidyl-2-one.

5. Procédé selon l'une des revendications 1 à 4, **caractérisé en ce que** le rapport molaire du composé (a) à l'alcool hétérocyclique (III) est compris entre 0,05 et 0,3 % et de préférence entre 0,05 et 0,15%.

6. Procédé selon l'une des revendications 1 à 5, **caractérisé en ce que** le catalyseur comprend en outre au moins un composé (b) choisi dans le groupe constitué par les alcoolates de magnésium, les alcoolates de sodium, les alcoolates de calcium, l'acétyl-acétonate de calcium, l'oxyde de calcium et l'hydroxyde de calcium.

7. Procédé selon la revendication 6, **caractérisé en ce que** le rapport molaire du composé (b) à l'alcool hétérocyclique (III) est compris entre 0,01 et 0,1 % et de préférence entre 0,01 et 0,05%.

8. Procédé selon l'une des revendications 1 à 7, **caractérisé en ce qu'**il est mis en oeuvre en présence d'au moins un inhibiteur de polymérisation choisi dans le groupe constitué par la phénotiazine, l'hydroquinone, l'éther monométhylique d'hydroquinone, le diterbutyl para-crésol, la paraphénylène diamine, le TEMPO et le di-tertiobutylcatéchol, ces composés étant éventuellement associés à des chélatants de métaux.

9. Procédé selon la revendication 8, **caractérisé en ce que** la teneur en inhibiteur de polymérisation est comprise entre 0,05 et 0,5% en poids par rapport au poids d'alcool hétérocyclique (III).

10. Procédé selon l'une des revendications 1 à 9, **caractérisé en ce qu'**il est mis en oeuvre en milieu anhydre.

11. Procédé selon l'une des revendications 1 à 10, **caractérisé en ce qu'**il est mis en oeuvre à une température comprise entre 70 et 120°C, de préférence entre 85 et 100°C.

12. Procédé selon l'une des revendications 1 à 11, **caractérisé en ce qu'**il est mis en oeuvre sous une pression comprise entre 0,3 et 1 bar.

13. Procédé selon la revendication 12, **caractérisé en ce qu'**il est mis en oeuvre sous bullage d'air.

14. Procédé selon l'une des revendications 1 à 13, **caractérisé en ce qu'**il est mis en oeuvre en présence d'un excès de réactif (II) ou de réactif (III).

15. Procédé selon la revendication 14, **caractérisé en ce que** le rapport molaire du (méth)acrylate (II) à l'alcool hétérocyclique (III) est supérieur à 1, de préférence compris entre 1,1 et 7, en particulier entre 2 et 5.

16. Composition comprenant un composé (I) obtenu par le procédé selon l'une des revendications 1 à 15 en solution dans le (méth)acrylate (II).

17. Composition selon la revendication 16, **caractérisée en ce que** sa coloration est inférieure à 100 APHA.

18. Utilisation d'une composition selon la revendication 16 ou 17, ou d'un composé obtenu par le procédé selon l'une des revendications 1 à 15, dans la préparation de polymères utilisables comme revêtements et adhésifs, dans le traitement du papier et des textiles, comme agent de traitement du cuir et dans la production de peintures à caractéristiques d'adhésion humide élevées.

## Claims

1. Process for the preparation of a compound (I) of formula: 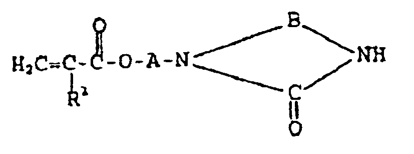 in which:
- R¹ is a hydrogen atom or a methyl group; and
- A and B represent, independently of each other, a straight- or branched-chain alkylene group having from 2 to 5 carbon atoms;
by reacting at least one (meth)acrylate (II) of formula: 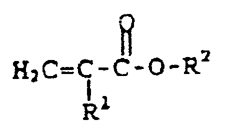
in which:
- R¹ has the abovementioned meaning; and
- R² is a straight- or branched-chain alkyl group having from 1 to 4 carbon atoms;
with a heterocyclic alcohol (III) of formula: 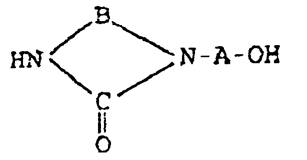
in which A and B have the abovementioned meanings;
in the presence of a catalyst comprising at least one compound (a) which is a chelate formed of lithium and of a 1,3-dicarbonyl compound.

2. Process according to Claim 1, in which the 1,3-dicarbonyl compound is:
- a β-ketonic acid ester, such as acetylacetic ester; or
- a 1,3-diketone, such as acetylacetone, 3-methylacetylacetone, benzoylacetone, dibenzoylmethane, 2,4-hexanedione, 3,5-heptanedione, 3-phenyl-acetylacetone, 4,4,4-trifluoro-1-phenyl-1,3-butanedione, 2,2,6,6-tetramethyl-3,5-heptanedione, 1,1,1-trifluoro-5,5-dimethyl-2,4-hexane-dione and 1,1,1-trifluoro-2,4-pentanedione.

3. Process according to Claim 2, **characterized in that** compound (a) is lithium acetylacetonate.

4. Process according to Claim 3, **characterized in that** reagent (II) is methyl acrylate or methacrylate and reagent (III) is 1-(2-hydroxyethyl)-imidazolidyl-2-one.

5. Process according to one of Claims 1 to 4, **characterized in that** the molar ratio of compound (a) to the heterocyclic alcohol (III) is between 0.05 and 0.3%, and preferably between 0.05 and 0.15%.

6. Process according to one of Claims 1 to 5, **characterized in that** the catalyst comprises, in addition, at least one compound (b) chosen from the group consisting of magnesium alkoxides, sodium alkoxides, calcium alkoxides, calcium acetylacetonate, calcium oxide and calcium hydroxide.

7. Process according to Claim 6, **characterized in that** the molar ratio of compound (b) to the heterocyclic alcohol (III) is between 0.01 and 0.1%, and preferably between 0.01 and 0.05%.

8. Process according to one of Claims 1 to 7, **characterized in that** it is carried out in the presence of at least one polymerization inhibitor chosen from the group consisting of phenothiazine, hydroquinone, the monomethyl ether of hydroquinone, di-tert-butyl-para-cresol, para-phenylenediamine, TEMPO and di-tert-butylcatechol, these compounds being optionally combined with metal chelators.

9. Process according to Claim 8, **characterized in that** the content of polymerization inhibitor is between 0.05 and 0.5% by weight relative to the weight of heterocyclic alcohol (III).

10. Process according to one of Claims 1 to 9, **characterized in that** it is carried out in an anhydrous medium.

11. Process according to one of Claims 1 to 10, **characterized in that** it is carried out at a temperature of between 70 and 120°C, preferably between 85 and 100°C.

12. Process according to one of Claims 1 to 11, **characterized in that** it is carried out at a pressure of between 0.3 and 1 bar.

13. Process according to Claim 12, **characterized in that** it is carried out under air bubbling.

14. Process according to one of Claims 1 to 13, **characterized in that** it is carried out in the presence of an excess of reagent (II) or of reagent (III).

15. Process according to Claim 14, **characterized in that** the molar ratio of the (meth)acrylate (II) to the heterocyclic alcohol (III) is greater than 1, preferably between 1.1 and 7, in particular between 2 and 5.

16. Composition comprising a compound (I) obtained by the process according to one of Claims 1 to 15 in solution in (meth)acrylate (II).

17. Composition according to Claim 16, **characterized in that** its colour is less than 100 APHA.

18. Use of a composition according to Claim 16 or 17, or obtained by the process according to one of Claims 1 to 15, in the preparation of polymers which can be used as coatings and adhesives, in the treatment of paper and of textiles, as agent for leather treatment and in the production of paints with high wet adhesion characteristics.

## Patentansprüche

1. Verfahren zur Herstellung einer Verbindung (I) der Formel: 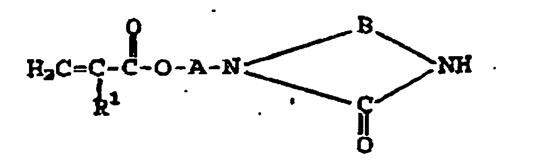 worin:
- R¹ Wasserstoff oder eine Methylgruppe ist; und
- A und B jeweils unabhängig voneinander für eine gerad- oder verzweigtkettige Alkylengruppe die 2 bis 5 Kohlenstoffatome aufweist, steht,
durch Umsetzung mindestens eines (Meth)acrylats (II) der Formel: 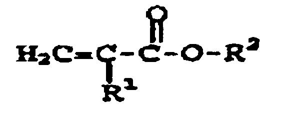 worin:
- R¹ die vorgenannte Bedeutung hat; und
- R² eine gerad- oder verzweigtkettige Alkylgruppe die 1 bis 4 Kohlenstoffatome aufweist, ist;
mit einem heterocyclischen Alkohol (III) der Formel: 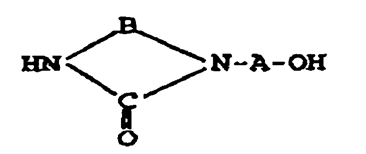 worin A und B die vorgenannten Bedeutungen haben; in Gegenwart eines Katalysators, der mindestens eine Verbindung (a) umfasst, die ein Chelat ist, das aus Lithium und einer 1,3-Dicarbonyl-Verbindung gebildet ist.

2. Verfahren nach Anspruch 1, worin die 1,3-Dicarbonylverbindung:
- β-Ketonsäureester, wie Acetessigsäureester, oder
- 1,3-Diketon ist, wie Acetylaceton, 3-Methylacetylaceton, Benzoylaceton, Dibenzoylmethan, 2,4-Hexandion, 3,5-Heptandion, 3-Phenylacetylaceton, 4,4,4-Trifluor-1-phenyl-1,3-butandion, 2,2,6,6-Tetramethyl-3,5-heptandion, 1,1,1-Trifluor-5,5-dimethyl-2,4-hexandion und 1,1,1-Trifluor-2,4-pentandion.

3. Verfahren nach Anspruch 2, **dadurch gekennzeichnet, dass** die Verbindung (a) Lithiumacetylacetonat ist.

4. Verfahren nach Anspruch 3, **dadurch gekennzeichnet, dass** der Reagens (II) Methylacrylat oder Methylmethacrylat ist und der Reagens (III) 1-(2-Hydroxyethyl)-imidazolidyl-2-on ist.

5. Verfahren nach einem der Ansprüche 1 bis 4, **dadurch gekennzeichnet, dass** das Molverhältnis der Verbindung (a) zum heterocyclischen Alkohol der Formel (III) zwischen 0,05% und 0,3% und bevorzugt zwischen 0,05 und 0,15% liegt.

6. Verfahren nach einem der Ansprüche 1 bis 5, **dadurch gekennzeichnet, dass** der Katalysator ferner mindestens eine Verbindung (b) umfasst, ausgewählt aus der Gruppe, bestehend aus Magnesiumalkoholaten, Natriumalkoholaten, Calciumalkoholaten, Calciumacetylacetonat, Calciumoxid und Calciumhydroxid.

7. Verfahren nach Anspruch 6, **dadurch gekennzeichnet, dass** das Molverhältnis der Verbindung (b) zum heterocyclischen Alkohol (III) zwischen 0,01% und 0,1% und bevorzugt zwischen 0,01 und 0,05% liegt.

8. Verfahren nach einem der Ansprüche 1 bis 7, **dadurch gekennzeichnet, dass** es in Gegenwart mindestens eines Polymerisationsinhibitors ausgeführt wird, ausgewählt aus der Gruppe, bestehend aus Phenothiazin, Hydrochinon, Hydrochinonmonomethylether, Diterbutylparacresol, Paraphenylendiamin, TEMPO und Di-tert-butylcatechol, wobei diese Verbindungen gegebenenfalls an Metallchelatbildner gebunden sind.

9. Verfahren nach Anspruch 8, **dadurch gekennzeichnet, dass** der Gehalt an Polymerisationsinhibitor zwischen 0,05 und 0,5 Gew.% im Verhältnis zum Gewicht des heterocyclischen Alkohols (III) liegt.

10. Verfahren nach einem der Ansprüche 1 bis 9, **dadurch gekennzeichnet, dass** es in einem wasserfreien Medium ausgeführt wird.

11. Verfahren nach einem der Ansprüche 1 bis 10, **dadurch gekennzeichnet, dass** es bei einer Temperatur zwischen 70 und 120°C, bevorzugt zwischen 85 und 100 °C, ausgeführt wird.

12. Verfahren nach einem der Ansprüche 1 bis 11, **dadurch gekennzeichnet, dass** es unter einem Druck zwischen 0,3 und 1 Bar ausgeführt wird.

13. Verfahren nach Anspruch 12, **dadurch gekennzeichnet, dass** es unter Luftzusammenballung ausgeführt wird.

14. Verfahren nach einem der Ansprüche 1 bis 13, **dadurch gekennzeichnet, dass** es in Gegenwart eines Überschusses an Reagens (II) oder Reagens (III) ausgeführt wird.

15. Verfahren nach Anspruch 14, **dadurch gekennzeichnet, dass** das Molverhältnis des Methacrylats (II) zum heterocyclischen Alkohol (III) größer als 1, bevorzugt zwischen 1,1 und 7, besonders bevorzugt zwischen 2 und 5 liegt.

16. Zusammensetzung, die eine Verbindung (I) umfasst, die durch das Verfahren nach einem der Ansprüche 1 bis 15 erhalten wurde, gelöst in (Meth)acrylat (II).

17. Zusammensetzung nach Anspruch 16, **dadurch gekennzeichnet, dass** ihre Färbung unter 100 APHA liegt.

18. Verwendung einer Zusammensetzung nach Anspruch 16 oder 17, oder einer Verbindung, die durch das Verfahren nach einem der Ansprüche 1 bis 15 erhalten wurde, zur Herstellung von Polymeren, die als Beschichtungen und Klebstoffe, zur Behandlung von Papier und Textilien, als Lederbehandlungsmittel und zur Produktion von Farben mit guten Feuchthaftungseigenschaften verwendbar sind.
